# FASCICULE DE BREVET EUROPEEN

(11) **EP 3 784 205 B1**
(45) Date de publication et mention de la délivrance du brevet: **23.07.2025**
(21) Numéro de dépôt: 19726061.5
(22) Date de dépôt: 24.04.2019
(51) Int. Cl.: A61K 8/36, A61Q 3/02, A61K 8/73

(54) **COMPOSITION COSMETIQUE DE VERNIS A ONGLES THIXOTROPE NON-AQUEUX RENFERMANT UN AGENT DURCISSEUR POUR LES ONGLES, UTILISATION ET PROCEDE DE MISE EN OEUVRE**
KOSMETISCHE ZUSAMMENSETZUNG EINES WASSERFREIEN THIXOTROPEN NAGELLACKS MIT EINEM NAGELHÄRTER, VERWENDUNG UND IMPLEMENTIERUNGSVERFAHREN
COSMETIC COMPOSITION OF NON-AQUEOUS THIXOTROPIC NAIL VARNISH CONTAINING A NAIL-HARDENING AGENT, USE AND IMPLEMENTATION METHOD

(30) Priorité: 25.04.2018 FR 1853597
(43) Date de publication de la demande: 03.03.2021
(73) Titulaire: Fiabila, 28130 Maintenon (FR)
(72) Inventeur: NOUGUEREDE, Olivier, 28130 HANCHES (FR)
(74) Mandataire: Ipsilon
(86) Numéro de dépôt international: PCT/FR2019/050966
(87) Numéro de publication internationale: WO 2019/207249

(56) Documents cités:
- EP-A1- 0 755 676
- FR-A1- 2 987 261
- "Leighton Denny Time Repair Gift Collection 12ml", 12 November 2017 (2017-11-12), XP002787702, Retrieved from the Internet <URL:https://www.lookfantastic.com/leighton-denny-time-repair-gift-collection-12ml/11344491.html> [retrieved on 20190108]

## Description

### DOMAINE DE L'INVENTION

L'invention concerne le domaine des compositions cosmétiques pour les ongles, plus particulièrement des compositions cosmétiques destinées à renforcer et durcir les ongles, ainsi que l'utilisation d'agent durcisseur des ongles, et le procédé associé.

### ETAT DE LA TECHNIQUE

Il est bien connu que les ongles, qui sont composés de kératine, sont parfois fragilisés, cassants, dédoublés ou fissurés. Outre la dégradation de l'aspect esthétique de ces ongles, il en résulte des désagréments importants pour la personne dans sa vie quotidienne, principalement lorsque ces défauts concernent les ongles des mains : accrochage dans les textiles, les vêtements, ou l'aggravation des fissurations lors de chaque préhension.

Jusqu'à maintenant, différentes solutions ont été apportées à ce problème, mais ces dernières sont soit imparfaites, soit génèrent d'autres inconvénients. On peut ainsi citer :
L'utilisation de formaldéhyde, dans des proportions pouvant aller jusqu'à 5 % en poids dans la composition cosmétique, est connue depuis longtemps. Cependant ce composé est maintenant très étroitement surveillé en raison de ses effets toxiques sur l'organisme, avec des effets cancérigènes avérés. Cette molécule est en outre un allergène puissant.

L'utilisation de glyoxal, un dialdéhyde, a été décrite dans le brevet GB 2 196 978 dès 1986. Ce produit est pourtant nocif par inhalation, irritant pour la peau et les yeux, et peut entraîner une sensibilisation de la peau.

Le citral (sous la forme d'un mélange de ses deux isomères géranial et néral), décrit dans le brevet EP 1 408 917, est certes également reconnu pour son action de durcisseur pour l'ongle, mais peut entraîner une sensibilisation par contact avec la peau et est classé parmi les allergènes détectés (selon la liste des substances allergènes citées dans le 7^{ème} amendement de la directive européenne 76/768/CEE). Il est donc de moins en moins utilisé en cosmétique. En outre, ces molécules comportent une double liaison, ce qui rend le citral sensible aux U.V., et donc sujet au jaunissement.

Dans le brevet EP 2 413 887 il a été proposé l'utilisation, en tant qu'agent durcisseur pour les ongles, de mono-aldéhyde de formule (I) : R-CHO, R étant un radical alkyle linéaire en C₅-C₁₂, ou un alcényle en C₅-C₁₂ à double liaison non conjuguée avec le groupement aldéhyde -CHO, n'appartenant pas à la liste des substances allergènes citées dans le 7^{ème} amendement de la directive européenne 76/768/CEE. Cependant ces aldéhydes, tel que l'hexanal, présentent une forte odeur, notamment dans le vernis sous forme liquide, ce qui limite leur usage et leur concentration dans lesdites compositions cosmétiques pour les ongles.

Cette liste d'inconvénients relatifs aux aldéhydes mis en œuvre dans des compositions cosmétiques pour les ongles a incité l'homme du métier à rechercher d'autres types de molécules non aldéhydiques pour durcir et renforcer la solidité des ongles humains.

Parmi les autres durcisseurs des ongles, le document US4,919,920 décrit l'incorporation d'iode ou de fluor sous forme d'ions dans une composition cosmétique aqueuse sans solvant organique, éventuellement en présence d'un polyol tel que la glycérine. Un tel usage n'est pas approprié aux vernis à ongles qui comprennent une phase importante de solvants organiques. L'iode peut aussi provoquer des allergies chez certains utilisateurs. Le document EP0755676 décrit l'utilisation d'acides monocarboxylique comme agent durcisseur.

Il subsiste donc un réel besoin de trouver un composé durcisseur des ongles qui palie les inconvénients précités et qui puisse être mis en œuvre dans des compositions cosmétiques variées, telles que des compositions de vernis, de sous-couche de vernis, ou de vernis pelable par exemple.

### BUTS DE L'INVENTION

Un premier but de l'invention est donc de proposer une composition de vernis à ongles incorporant un composé destiné à renforcer la structure de la kératine de l'ongle en la durcissant, ne présentant pas les inconvénients mentionnés ci-dessus. Un autre but de l'invention est de proposer un composé qui ne soit pas sensible au vieillissement, et notamment au jaunissement, aux concentrations auxquelles il est efficace en tant que durcisseur pour les ongles, dans une composition cosmétique de vernis.

Encore un but de l'invention est de proposer un composé durcisseur pour les ongles ne présentant pas d'odeur gênante pour l'utilisateur, aux concentrations auxquelles il est efficace en tant que durcisseur pour les ongles, dans une composition cosmétique de vernis.

Un autre but de l'invention est de proposer une composition de vernis à ongles thixotrope, c'est-à-dire fluide lors de son application mais présentant une viscosité supérieure au repos, incorporant un composé durcisseur pour les ongles qui ne modifie pas les propriétés thixotropes dudit vernis.

### DESCRIPTION DE L'INVENTION

A cet effet, la présente invention propose une composition cosmétique de vernis à ongles thixotrope non-aqueux renfermant un agent filmogène comprenant la nitrocellulose, au moins un solvant organique, et un agent thixotropique, caractérisée en ce qu'elle renferme de l'acide sorbique ou l'un de ses sels à une concentration inférieure à 1 % en poids et en ce que l'agent thixotropique, présent à une concentration comprise entre 0,1 et 4 % en poids de la composition, est une argile modifiée avec une amine quaternaire, neutralisée par un acide fort à une concentration inférieure à 0,1 % en poids de la composition.

La composition de vernis est de type solvantaire, c'est-à-dire à base majoritairement (renfermant plus de 50 % en poids) de solvants organiques. Par non-aqueux, ou essentiellement non-aqueux, on entend ici une composition de vernis dont la teneur en eau ne dépasse pas 3 % en poids, de préférence inférieure ou égale à 2 % en poids, et de préférence encore inférieure ou égale à 1 %.

L'acide sorbique n'est pas un produit toxique, ni allergène : en effet cette molécule est fréquemment utilisée en tant que conservateur (E200) dans le domaine agro-alimentaire, en tant qu'agent anti-microbien pour inactiver ou inhiber la croissance des moisissures et des bactéries dans des compositions alimentaires renfermant une fraction aqueuse importante.

En outre, cette molécule, malgré la présence de deux doubles liaisons, ne génère pas de jaunissement dans le temps ni d'odeur incommodante pour l'utilisateur à des concentrations inférieures ou égales à 1 % en poids. Ainsi, l'acide sorbique peut être mis en œuvre dans une composition cosmétique pour les ongles. De plus l'acide sorbique et ses sels ont l'avantage de ne pas présenter de danger pour la santé (sensibilisation, allergie, toxicité, ni effet cancérigène, au contraire de certains actifs de l'art antérieur).

De manière avantageuse, ladite composition cosmétique renferme une concentration en acide sorbique ou l'un de ses sels inférieure ou égale à 0,5 % en poids. De manière encore avantageuse, la composition cosmétique renferme une concentration en acide sorbique ou l'un de ses sels comprise entre 0,01% et 0,5 % en poids, de préférence encore comprise entre 0,05 % et 0,1 % en poids.

L'agent thixotropique de la composition de vernis selon l'invention est une argile modifiée avec une amine quaternaire, l'argile étant choisie de préférence parmi la bentonite ou l'hectorite. Or ces agiles comportant des greffages d'amines quaternaires sont particulièrement sensibles à la présence d'acides, modifiant de ce fait leur comportement thixotrope. Il s'est cependant avéré que, même sous la forme d'acide libre, l'acide sorbique n'avait pas d'effet néfaste sur la stabilité de la composition de vernis, ni sur son caractère thixotrope.

La concentration en agent thixotropique est avantageusement comprise entre 0,5 et 3 % en poids, de préférence entre 1 et 2 % en poids de la composition.

L'acide fort neutralisant l'argile modifiée avec une amine quaternaire est avantageusement l'acide phosphorique, de préférence à une concentration inférieure ou égale à 0,05 % en poids, de préférence inférieure ou égale à 0,03 % en poids de la composition. A de telles concentrations, l'acide phosphorique, tout en permettant l'action thixotrope de l'argile, ne décolore pas les pigments pouvant être présents dans la composition de vernis.

De manière avantageuse, la concentration en nitrocellulose est comprise entre 8 et 30 % en poids, de préférence entre 10 et 25 % en poids, de préférence encore entre 12 et 20 % en poids de la composition. Les autres dérivés cellulosiques en tant qu'agent filmogène sont écartés, en raison de leur mauvaise adhésion sur l'ongle.

La composition cosmétique selon l'invention peut être une composition cosmétique sous la forme d'un vernis à ongle coloré ou incolore, d'une sous-couche de vernis, ou d'un vernis pelable. Il est bien entendu possible d'incorporer à ces compositions cosmétiques des colorants, des pigments et/ou nacres pour réaliser des vernis à ongles de couleurs et d'aspects différents.

Les inventeurs ont constaté de manière surprenante que l'acide sorbique présent dans une telle composition de vernis à ongles thixotrope confère des propriétés de renforcement et de durcissement auxdits ongles. Dans la composition cosmétique de vernis à ongles selon l'invention l'acide sorbique ou l'un de ses sels est ainsi avantageusement utilisé en tant qu'agent durcisseur pour les ongles.

Cette utilisation est non-thérapeutique. Certains acides organiques, ou leurs sels, étant déjà connus (notamment du document US 2008/0193508) comme agents antifongiques, notamment contre des mycoses des ongles, c'est-à-dire pour un usage thérapeutique.

La présente invention concerne donc également l'utilisation d'acide sorbique, ou de l'un de ses sels, en tant qu'agent durcisseur pour les ongles dans une composition cosmétique telle que décrite ci-dessus appliquée sur des ongles exempts d'infection fongique.

La composition peut renfermer une concentration en acide sorbique ou en l'un de ses sels inférieure ou égale à 0,5 % en poids, et avantageusement une concentration en acide sorbique ou en l'un de ses sels comprise entre 0,01% et 0,5 % en poids, de préférence encore comprise entre 0,05 % et 0,1 % en poids.

En effet, il a été également constaté que les propriétés durcissantes de l'acide sorbique sur les ongles sont présentes, même à des concentrations très faibles, inférieures ou égales à 0,1 % en poids (voir plus loin les exemples 1-2).

Selon la présente invention, l'acide sorbique en tant qu'agent durcisseur pour les ongles est incorporé dans une composition cosmétique filmogène, tel qu'un vernis à ongles coloré ou incolore, une sous-couche de vernis ou une vernis pelable. Les compositions cosmétiques filmogènes, telles que les vernis, sont préférées car elles peuvent rester durablement sur l'ongle.

L'acide sorbique appliqué sur les ongles sains, c'est-à-dire exempts d'infection fongique, peut néanmoins être actif à plusieurs niveaux sur les défauts des ongles : ongles fissurés, ongles fins, ongles cassants, ongles dédoublés, dureté de l'ongle, fragilité de l'ongle, sans provoquer de réactions allergiques.

La présente invention concerne également un procédé pour renforcer et durcir des ongles, exempts d'infection fongique, notamment des ongles fissurés, fins, cassants, mous ou dédoublés, consistant à appliquer de manière topique sur lesdits ongles, une composition cosmétique de vernis à ongles telle que décrite ci-dessus, renfermant l'acide sorbique ou l'un de ses sels.

Les exemples suivants permettent d'illustrer, de manière non limitative, la présente invention. Dans l'ensemble des formulations, la concentration des différents constituants est exprimée en % en poids du poids total de la composition cosmétique.

### EXEMPLES

Différentes compositions cosmétiques sous la forme de vernis à ongle de type vernis à base solvant organique ont été préparées et appliquées sur des ongles. Les tests ci-après ont été réalisés sur ces vernis :
- Brillance : Sur une plaque de type Leneta, 100µm de vernis a été appliqué. Après séchage, la brillance a été mesurée à un angle d'indice de 60°, au moyen d'un Brillancemètre MINOLTA 268.

- Flexibilité : Sur une plaque en aluminium recouverte d'un vernis de 300µm humide, on réalise un emboutissage lent et on mesure la profondeur de la pénétration (ISO1520).
- Dureté du vernis : a été mesurée à l'aide d'un pendule de Persoz sur une plaque de verre recouverte d'un vernis d'épaisseur de 100µm humide, après séchage à température ambiante, selon la norme ISO1522.
- Adhérence : un « Cross hatch test » est réalisé sur plaque de verre. La note 0 correspond à une absence de perte d'adhésion. La note 5 correspond à une perte totale d'adhésion.
- Extrait sec : Dans une coupelle, on verse entre 0,5 et 1g de vernis. Cette plaque est ensuite mise à l'étuve durant 3 heures à 100°C, puis pesée pour calculer l'extrait sec du vernis.
- Stabilité au jaunissement : Les vernis sont conditionnés dans des flacons en verre et stockés dans une étuve à 50°C durant 1 mois. La couleur du vernis est ensuite comparée à un vernis de référence ne contenant pas d'agent durcisseur.
- Stabilité à la sédimentation : Une composition de vernis teintée contenant de la nacre et une solution colorante est préparée et est placée à l'étuve durant 1 mois à 45°C. Une observation visuelle est réalisée pour vérifier si la nacre a sédimenté ou non.
- Stabilité des teintes : Un vernis teinté contenant un mélange des pigments suivants : dioxyde de titane, Red 34 et Bleu ferric ferrocyanide, est placé à l'étuve à 45°C durant 30 jours. On observe la stabilité de cette teinte et plus spécifiquement l'apparition ou non de synérèse.
- Odeur : L'odeur du vernis est comparée à celle d'un vernis ne contenant pas d'agent durcisseur.
- Viscosité : La viscosité du produit est mesurée à 25°C à l'aide d'un viscosimètre Brookfield. Les mesures sont prises après une rotation d'une minute à 6 tr/min, puis après une minute à 60 tr/min, enfin après une minute à 6 tr/min. Les valeurs sont données en mPa.s

### Exemples 1 à 3, exemple comparatif 4

Des compositions cosmétiques sous la forme de vernis à ongle de type vernis à base solvant organique ont été préparées à partir des constituants présentés dans le tableau 1 suivant. Ces compositions renfermaient des concentrations variables en acide sorbique, entre 0,05 et 1 % en poids.

**Tableau 1**

| **Composition** | **Ex. 1** | **Ex. 2** | **Ex. 3** | **Ex. Comp. 4** |
|---|---|---|---|---|
| Acétate de butyle | 42,52 | 42,47 | 42,07 | 41,57 |
| Acétate d'éthyle | 20,6 | 20,6 | 20,6 | 20,6 |
| Nitrocellulose | 14 | 14 | 14 | 14 |
| Résine polyester | 9,5 | 9,5 | 9,5 | 9,5 |
| Acétyltributyl citrate | 6 | 6 | 6 | 6 |
| Alcool isopropylique | 6 | 6 | 6 | 6 |
| Stéaralkonium bentonite | 1,3 | 1,3 | 1,3 | 1,3 |
| Acide phosphorique | 0,03 | 0,03 | 0,03 | 0,03 |
| Acide sorbique | 0,05 | 0,1 | 0,5 | 1 |

| **Résultats** | | | | |
|---|---|---|---|---|
| Stabilité | OK | OK | OK | Jaunissement |
| Dureté du vernis (s) | 240 | 248 | 243 | 235 |
| Viscosité (mPa.s) | | | | |
| 6rpm | 3300 | 3250 | 3290 | 3320 |
| 60rpm | 1030 | 1020 | 1040 | 1030 |
| 6rpm | 2100 | 2090 | 2120 | 2140 |
| Stabilité teintes | Pas de synérèse | Pas de synérèse | Pas de synérèse | Pas de synérèse |
| Adhésion | 0 | 0 | 0 | 0 |

La résine polyester du tableau 1 ci-dessus est un copolymère acide adipique/néopentyl glycol/anhydride triméllitique dilué à 70 % d'extrait sec dans de l'acétate de butyle.

La nitrocellulose est mouillée à 70% dans de l'isopropanol.

Ces compositions cosmétiques se présentent sous la forme d'un vernis, incolore et transparent après application sur l'ongle, présentant une teneur en extrait sec égal à 24% en poids (mesurée à 100 °C). Une ou plusieurs matières colorantes choisies parmi les pigments, les colorants solubles et les particules décoratives, telles que les nacres et les paillettes, peuvent être ajoutées.

On note que la quantité maximum d'acide sorbique pouvant être incorporée au vernis, sans provoquer de jaunissement, est inférieure à 1 % en poids.

De plus, l'acide sorbique à ces concentrations inférieures à 1 % en poids, ne présente pas une odeur désagréable pour l'utilisateur. Il n'a pas non plus d'influence sur la viscosité de la composition de vernis, ni sur sa stabilité.

### Exemples comparatifs 5-6 et 9, et exemple 7 - Tests in vivo :

Quatre compositions de vernis selon l'exemple 1 renfermant chacune 0,05 % en poids d'agent durcisseur et une composition de vernis de référence sans agent durcisseur ont fait chacune l'objet de tests in vivo sur des panels de 30 personnes âgées d'au moins 20 ans pendant trois semaines pour en évaluer l'efficacité et la tolérance. Aucune des personnes testées ne présentait de lésion au niveau des zones cibles (ici le pourtour des ongles des mains), ni d'affection dermatologique, ni d'infection fongique des ongles. Le vernis a été appliqué dans des conditions normales d'emploi (à savoir une application tous les trois jours, après avoir retiré le vernis précédent).

La composition comparative 5 renfermait 0,05 % d'hexanal, la composition comparative 6 renfermait 0,05 % en poids de citral, la composition comparative 9 0,05 % en poids d'acide caproïque et la composition 7 selon la présente invention 0,05 % en poids d'acide sorbique. La composition de vernis 8 était sans agent durcisseur (composition de référence).

Les compositions respectives ainsi que les résultats des tests physiques et observés par les panelistes sont regroupés dans le tableau 2 ci-après.

**Tableau 2**

| **Compositions** | **Ex.5 Comp.** | **Ex. 6 Comp.** | **Ex. 7** | **Ex. 8 (Réf.)** | **Ex. 9 Comp.** |
|---|---|---|---|---|---|
| Acétate de butyle | 42,53 | 42,52 | 42,52 | 42,57 | 42,52 |
| Acétate d'éthyle | 20,6 | 20,6 | 20,6 | 20,6 | 20,6 |
| Nitrocellulose | 14 | 14 | 14 | 14 | 14 |
| Résine polyester | 9,5 | 9,5 | 9,5 | 9,5 | 9,5 |
| Acétyltributyl citrate | 6 | 6 | 6 | 6 | 6 |
| Alcool isopropylique | 6 | 6 | 6 | 6 | 6 |
| Stéaralkonium bentonite | 1,3 | 1,3 | 1,3 | 1,3 | 1,3 |
| Acide phosphorique | 0,03 | 0,03 | 0,03 | 0,03 | 0,03 |
| Hexanal | 0,05 | - | - | - | - |
| Citral | - | 0,05 | - | - | - |
| Acide sorbique | - | - | 0,05 | - | - |
| Acide caproïque | | | | | 0,05 |

| **Résultats des tests** | | | | | |
|---|---|---|---|---|---|
| Brillance | 86,7 | 87,1 | 87 | 86,9 | |
| Flexibilité | 4,5 | 4,4 | 4,6 | 4,4 | 4,4 |
| Dureté du vernis(s) | 236 | 233 | 240 | 238 | 235 |
| Adhérence | 0 | 0 | 0 | 0 | 0 |
| Extrait sec | 28,1 | 28,3 | 27,9 | 28 | 28,1 |
| Stabilité | OK | OK | OK | Référence | OK |
| Odeur | Forte | Légère et agréable | Absence | Référence | Absence |

| **Dureté de l'ongle** | | | | | |
|---|---|---|---|---|---|
| Nb personnes répondant Oui | 23 | 18 | 25 | 12 | 14 |
| Pourcentage | 76,7 | 60,0 | 83,3 | 40,0 | 46,7 |

Aucun signe clinique d'intolérance n'a été constaté, ni ressenti par les personnes testées. Aucun symptôme d'allergie n'a été relevé.

En outre, plus de 83 % des sujets ont trouvé une amélioration globale de la qualité et la dureté de leurs ongles. Ces résultats démontrent les propriétés in vivo de durcisseur de l'ongle de l'acide sorbique, et ceci à une faible concentration (0,05 % en poids) dans la composition de vernis.

### Exemples 11 et 12 - Exemples comparatifs 10 et 13 : Stabilité à la sédimentation et tenue de la couleur

Pour vérifier la stabilité à la sédimentation, ont été ajoutées à la composition de vernis de base selon la revendication 2, deux matières colorantes:
d'une part, une **Nacre** : Mica titane commercialisé sous le nom Flamenco gold, 6-48µm, par la société BASF,
et d'autre part le **Pigment Red 34** : Pigment organique commercialisé par la société SUN Chemical.

Ce pigment a été broyé pour pouvoir être incorporé. Le protocole de broyage incluait le mélange des solvants avec le pigment, l'agitation pour désagglomérer le pigment, puis l'incorporation de la nitrocellulose. Une fois cette dernière dissoute, le plastifiant a été incorporé sous agitation, poursuivie pendant 20 minutes. Le mélange obtenu a ensuite été broyé dans un broyeur à bille.

Le pigment Red 34 a été ainsi broyé avec les constituants suivants (% en poids) :

**Tableau 3**

| | |
|---|---|
| Acétate de butyle | 30 |
| Acétate d'éthyle | 30 |
| Plastifiant (A.T.C.) | 10 |
| Red 34 | 10 |
| Nitrocellulose | 20 |

La teinte suivante a été réalisée sur chacun des essais décrits ci-après :

| | |
|---|---|
| Composition de vernis de base | 95 % en poids |
| Solution contenant le Red 34 | 3 % en poids |
| Mica titane (Flamenco gold, 6-48µm, de la société BASF) | 2 % en poids |

La stabilité des compositions ainsi préparées a été évaluée observation visuelle après conservation un mois à 45°C.

**Tableau 4**

| **Composition** | **Ex. 2** | **Ex. 10 (comp.)** | **Ex. 11** | **Ex. 12** | **Ex. 13 (comp.)** |
|---|---|---|---|---|---|
| Acétate de butyle | 42,47 | 42,52 | 42,49 | 42,45 | 42,4 |
| Acétate d'éthyle | 20,6 | 20,6 | 20,6 | 20,6 | 20,6 |
| Nitrocellulose | 14 | 14 | 14 | 14 | 14 |
| Résine polyester | 9,5 | 9,5 | 9,5 | 9,5 | 9,5 |
| Acétyl tributyl citrate | 6 | 6 | 6 | 6 | 6 |
| Alcool isopropylique | 6 | 6 | 6 | 6 | 6 |
| Stéaralkonium bentonite | 1,3 | 1,3 | 1,3 | 1,3 | 1,3 |
| Acide phosphorique | 0,03 | | 0,01 | 0,05 | 0.1 |
| Acide citrique | | 0.03 | | | |
| Acide sorbique | 0,1 | 0.1 | 0,1 | 0.1 | 0.1 |

| **Résultats** | | | | | |
|---|---|---|---|---|---|
| Stabilité au jaunissement | OK | OK | OK | OK | OK |
| Dureté du vernis (s) | 248 | 242 | 240 | 240 | 237 |
| Viscosité (mPa.s) | | | | | |
| 6rpm | 3250 | 1400 | 1890 | 4090 | 5630 |
| 60rpm | 1020 | 980 | 1010 | 1080 | 1120 |
| 6rpm | 2090 | 1230 | 1460 | 2530 | 3250 |
| Adhésion | 0 | 0 | 0 | 0 | 0 |
| Stabilité de la teinte décrite ci-dessus | Stable | Sédimentation de la nacre | Stable | Stable | Décoloration de la teinte |

### Conclusions :

Une sédimentation est observée en présence d'acide citrique en remplacement de l'acide phosphorique.

L'acide phosphorique à 0,1% en poids dans le vernis décolore le pigment Red 34.

## Revendications

1. Composition cosmétique de vernis à ongles thixotrope non-aqueux renfermant un agent filmogène comprenant la nitrocellulose, au moins un solvant organique, et un agent thixotropique, **caractérisée en ce qu'**elle renferme de l'acide sorbique ou l'un de ses sels à une concentration inférieure à 1 % en poids et **en ce que** l'agent thixotropique, présent à une concentration comprise entre 0,1 et 4 % en poids de la composition, est une argile modifiée avec une amine quaternaire, neutralisée par un acide fort à une concentration inférieure à 0,1 % en poids de la composition.

2. Composition cosmétique de vernis à ongles selon la revendication 1, **caractérisée en ce qu'**elle renferme une concentration en acide sorbique ou l'un de ses sels inférieure ou égale à 0,5 % en poids, de préférence comprise entre 0,01 % et 0,5 % en poids, de préférence encore comprise entre 0,05 % et 0,1 % en poids.

3. Composition cosmétique de vernis à ongles selon l'une des revendications 1 ou 2, **caractérisée en ce que** l'agent thixotropique est une argile modifiée avec une amine quaternaire, l'argile étant choisie parmi la bentonite ou l'hectorite.

4. Composition cosmétique de vernis à ongles selon l'une quelconque des revendications précédentes, **caractérisée en ce que** la concentration en agent thixotropique est comprise entre 0,5 et 3 % en poids, de préférence entre 1 et 2 % en poids de la composition.

5. Composition cosmétique de vernis à ongles selon l'une quelconque des revendications précédentes, **caractérisée en ce que** l'acide fort neutralisant l'argile modifiée avec une amine quaternaire est l'acide phosphorique, de préférence à une concentration inférieure ou égale à 0,05 % en poids, de préférence inférieure ou égale à 0,03 % en poids de la composition.

6. Composition cosmétique de vernis à ongles selon l'une quelconque des revendications précédentes, **caractérisée en ce que** la concentration en nitrocellulose est comprise entre 8 et 30 % en poids, de préférence entre 10 et 25 % en poids, de préférence encore entre 12 et 20 % en poids de la composition.

7. Composition cosmétique de vernis à ongles selon l'une quelconque des revendications précédentes, **caractérisée en ce que** l'acide sorbique ou l'un de ses sels est utilisé en tant qu'agent durcisseur pour les ongles.

8. Composition cosmétique de vernis à ongles selon l'une quelconque des revendications précédentes, **caractérisée en ce que** la composition est une composition cosmétique sous la forme d'un vernis à ongle coloré ou incolore, d'une sous-couche de vernis, ou d'un vernis pelable.

9. Utilisation d'acide sorbique, ou de l'un de ses sels, en tant qu'agent durcisseur des ongles, à une concentration inférieure à 1 % en poids, dans une composition cosmétique selon l'une quelconque des revendications précédentes appliquée sur des ongles exempts d'infection fongique.

10. Utilisation selon la revendication 9, **caractérisée en ce que** la composition cosmétique renferme une concentration en acide sorbique ou en l'un de ses sels inférieure ou égale à 0,5 % en poids.

11. Utilisation selon l'une des revendications 9 ou 10, **caractérisée en ce que** la composition cosmétique renferme une concentration en acide sorbique ou en l'un de ses sels comprise entre 0,01% et 0,5 % en poids, de préférence encore comprise entre 0,05 % et 0,1 % en poids.

12. Procédé pour renforcer et durcir des ongles exempts d'infection fongique, notamment des ongles fissurés, fins, cassants, mous ou dédoublés, consistant à appliquer de manière topique sur lesdits ongles une composition cosmétique de vernis selon l'une quelconque des revendications 1 à 8.

## Patentansprüche

1. Nichtwässrige thixotrope kosmetische Nagellackzusammensetzung, enthaltend einen Filmbildner, der Nitrocellulose umfasst, mindestens ein organisches Lösungsmittel und ein Thixotropiermittel, **dadurch gekennzeichnet, dass** sie Sorbinsäure oder eines ihrer Salze in einer Konzentration von weniger als 1 Gew.-% enthält und dass es sich bei dem Thixotropiermittel, das in einer Konzentration zwischen 0,1 und 4 Gew.-% der Zusammensetzung vorliegt, um einen mit einem quartären Amin modifizierten Ton, der mit einer starken Säure bis zu einer Konzentration von weniger als 0,1 Gew.-% der Zusammensetzung neutralisiert ist, handelt.

2. Kosmetische Nagellackzusammensetzung nach Anspruch 1, **dadurch gekennzeichnet, dass** sie eine Konzentration von Sorbinsäure oder einem ihrer Salze kleiner oder gleich 0,5 Gew.-%, vorzugsweise zwischen 0,01 Gew.-% und 0,5 Gew.-%, weiter bevorzugt zwischen 0,05 % und 0,1 Gew.-%, enthält.

3. Kosmetische Nagellackzusammensetzung nach einem der Ansprüche 1 oder 2, **dadurch gekennzeichnet, dass** es sich bei dem Thixotropiermittel um einen mit einem quartären Amin modifizierten Ton handelt, wobei der Ton aus Bentonit oder Hectorit ausgewählt ist.

4. Kosmetische Nagellackzusammensetzung nach einem der vorhergehenden Ansprüche, **dadurch gekennzeichnet, dass** die Konzentration des Thixotropiermittels zwischen 0,5 und 3 Gew.-%, vorzugsweise zwischen 1 und 2 Gew.-%, der Zusammensetzung liegt.

5. Kosmetische Nagellackzusammensetzung nach einem der vorhergehenden Ansprüche, **dadurch gekennzeichnet, dass** es sich bei der den mit einem quartären Amin modifizierten Ton neutralisierenden starken Säure um Phosphorsäure handelt, vorzugsweise in einer Konzentration kleiner oder gleich 0,05 Gew.-%, vorzugsweise kleiner oder gleich 0,03 Gew.-%, der Zusammensetzung.

6. Kosmetische Nagellackzusammensetzung nach einem der vorhergehenden Ansprüche, **dadurch gekennzeichnet, dass** die Konzentration von Nitrocellulose zwischen 8 und 30 Gew.-%, vorzugsweise zwischen 10 und 25 Gew.-%, weiter bevorzugt zwischen 12 und 20 Gew.-%, der Zusammensetzung liegt.

7. Kosmetische Nagellackzusammensetzung nach einem der vorhergehenden Ansprüche, **dadurch gekennzeichnet, dass** die Sorbinsäure oder eines ihrer Salze als Härtungsmittel für die Nägel verwendet wird.

8. Kosmetische Nagellackzusammensetzung nach einem der vorhergehenden Ansprüche, **dadurch gekennzeichnet, dass** es sich bei der Zusammensetzung um eine kosmetische Zusammensetzung in Form eines farbigen oder farblosen Nagellacks, einer Lackunterschicht oder eines abziehbaren Lacks handelt.

9. Verwendung von Sorbinsäure oder einem ihrer Salze in einer Konzentration von weniger als 1 Gew.-% als Nagelhärtungsmittel in einer kosmetischen Zusammensetzung nach einem der vorhergehenden Ansprüche, die auf Nägel, die frei von Pilzinfektion sind, aufgebracht wird.

10. Verwendung nach Anspruch 9, **dadurch gekennzeichnet, dass** die kosmetische Zusammensetzung eine Konzentration von Sorbinsäure oder einem ihrer Salze kleiner oder gleich 0,5 Gew.-% enthält.

11. Verwendung nach einem der Ansprüche 9 oder 10, **dadurch gekennzeichnet, dass** die kosmetische Zusammensetzung eine Konzentration von Sorbinsäure oder einem ihrer Salze zwischen 0,01 und 0,5 Gew.-%, weiter bevorzugt zwischen 0,05 und 0,1 Gew.-%, enthält.

12. Verfahren zum Festigen und Härten von Nägeln, die frei von Pilzinfektion sind, insbesondere rissigen, dünnen, brüchigen, weichen oder gespaltenen Nägeln, das darin besteht, dass man auf die Nägel topisch eine kosmetische Lackzusammensetzung nach einem der Ansprüche 1 bis 8 aufbringt.

## Claims

1. Nonaqueous thixotropic cosmetic nail varnish composition containing a film-forming agent comprising nitrocellulose, at least one organic solvent, and a thixotropic agent, **characterized in that** said composition contains sorbic acid or one of its salts at a concentration of less than 1% by weight, and **in that** the thixotropic agent, present at a concentration of between 0.1% and 4% by weight of the composition, is a clay modified with a quaternary amine, neutralized by a strong acid at a concentration of less than 0.1% by weight of the composition.

2. Cosmetic nail varnish composition according to Claim 1, **characterized in that** it contains a concentration of sorbic acid or one of its salts of less than or equal to 0.5% by weight, preferably of between 0.01% and 0.5% by weight, more preferably of between 0.05% and 0.1% by weight.

3. Cosmetic nail varnish composition according to either of Claims 1 and 2, **characterized in that** the thixotropic agent is a clay modified with a quaternary amine, the clay being chosen from bentonite or hectorite.

4. Cosmetic nail varnish composition according to any one of the preceding claims, **characterized in that** the concentration of thixotropic agent is between 0.5% and 3% by weight, preferably between 1% and 2% by weight, of the composition.

5. Cosmetic nail varnish composition according to any one of the preceding claims, **characterized in that** the strong acid which neutralizes the clay modified with a quaternary amine is phosphoric acid, preferably at a concentration of less than or equal to 0.05% by weight, preferably of less than or equal to 0.03% by weight, of the composition.

6. Cosmetic nail varnish composition according to any one of the preceding claims, **characterized in that** the concentration of nitrocellulose is between 8% and 30% by weight, preferably between 10% and 25% by weight, more preferably between 12% and 20% by weight, of the composition.

7. Cosmetic nail varnish composition according to any one of the preceding claims, **characterized in that** the sorbic acid or one of its salts is used as a hardening agent for the nails.

8. Cosmetic nail varnish composition according to any one of the preceding claims, **characterized in that** the composition is a cosmetic composition in the form of a coloured or colourless nail varnish, of a varnish base coat, or of a peelable varnish.

9. Use of sorbic acid, or of one of its salts, as a nail-hardening agent, at a concentration of less than 1% by weight, in a cosmetic composition according to any one of the preceding claims applied to nails free from fungal infection.

10. Use according to Claim 9, **characterized in that** the cosmetic composition contains a concentration of sorbic acid or of one of its salts of less than or equal to 0.5% by weight.

11. Use according to either of Claims 9 and 10, **characterized in that** the cosmetic composition contains a concentration of sorbic acid or of one of its salts of between 0.01% and 0.5% by weight, more preferably of between 0.05% and 0.1% by weight.

12. Method for strengthening and hardening nails free from fungal infection, in particular cracked, thin, brittle, soft or split nails, consisting in topically applying to said nails a cosmetic varnish composition according to any one of Claims 1 to 8.
